# DEMANDE DE BREVET EUROPEEN

(11) **EP 1 151 745 A1**
(43) Date de publication de la demande: **07.11.2001**
(21) Numéro de dépôt: 01401039.1
(22) Date de dépôt: 24.04.2001
(51) Int. Cl.: A61K 7/48, A61K 7/00

(54) **Composition sous forme d'émulsion eau-dans-huile et ses utilisations cosmétiques**

(30) Priorité: 05.05.2000 FR 0005796
(71) Demandeur: L'OREAL, 75008 Paris (FR)
(72) Inventeur: Sonneville-Aubrun, Odile, 92160 Antony (FR); Simonnet, Jean-Thierry, 75011 Paris (FR)
(74) Mandataire: Rasson, Catherine

(57) **Abrégé**

L'invention se rapporte à une composition sous forme d'une émulsion eau dans huile, contenant au moins 80 % de phase aqueuse et au moins un glycoglycéride.

Le glycoglycéride peut être obtenu par synthèse ou par extraction, et il peut être notamment un galactolipide tel que par exemple le digalactosyl diglycéride ou le monogalactosyl diglycéride.

La composition selon l'invention apporte un grand effet de fraîcheur lors de l'application sur la peau.

Cette composition est utilisable notamment dans les domaines cosmétique et/ou dermatologique.

## Description

L'invention se rapporte à une composition se présentant sous forme d'une émulsion eau-dans-huile (E/H) comportant une forte teneur en eau et contenant au moins un glycoglycéride, et à ses utilisations, en particulier dans les domaines cosmétique et/ou dermatologique.

Dans les domaines cosmétique ou dermatologique, il est courant d'utiliser des compositions constituées d'une émulsion eau-dans-huile (E/H) comportant une phase aqueuse dispersée dans une phase huileuse. Ces émulsions comportent une phase continue huileuse et permettent donc de former à la surface de la peau un film lipidique qui prévient la perte d'eau transépidermique et protège la peau des agressions extérieures. Ces émulsions sont particulièrement appropriées pour protéger et nourrir la peau, et en particulier pour traiter les peaux sèches. En outre, le film lipidique formé à la surface de la peau peut aussi augmenter la rémanence des filtres solaires. Ces émulsions ont aussi l'avantage de permettre la protection et le transport des actifs hydrophiles sensibles à l'oxydation.

Les émulsions inverses, c'est-à-dire eau-dans-huile, sont habituellement stabilisées par des tensioactifs lipophiles qui sont le plus couramment choisis parmi les dérivés alkylés de polyglycérol, les dérivés alkylés polyoxyéthylénés, les dérivés alkylés de sorbitane, les sels métalliques d'acides gras. Généralement, la teneur en phase aqueuse de ces émulsions doit rester inférieure à 80 % du poids de la composition, et le taux de tensioactif doit être relativement élevé, pour que les émulsions obtenues aient une stabilité acceptable, c'est-à-dire sans déphasage ni de relargage d'huile et/ou d'eau au cours du temps.

Le plus souvent, en raison du taux de tensioactif élevé et de la teneur en eau limitée, les formules obtenues sont grasses, lourdes et collantes. De plus, le taux de tensioactif peut induire des problèmes de tolérance, notamment chez les sujets à peau sensible.

Par ailleurs, pour améliorer la stabilité de ces émulsions, il est généralement nécessaire d'augmenter la viscosité de la phase huileuse en ajoutant un agent structurant, et par exemple soit des cires comme décrit par exemple dans le document DE-A-3430256, soit des gélifiants huileux comme décrit par exemple dans le document EP-A-795321, soit des argiles modifiées comme décrit par exemple dans le document EP-A-331833. Toutefois, du fait de l'ajout d'un agent structurant, les compositions obtenues manquent de fraîcheur et de légèreté. Elles donnent une sensation de gras lors de l'application sur la peau.

Pour pallier à ces inconvénients des émulsions inverses à forte teneur en phase aqueuse et à faible taux de tensioactif, on a utilisé des tensioactifs siliconés tels que les alkylméthicones copolyols et les alkyldiméthicones copolyols comme décrit dans le document DE-A-19826750, ou bien tels que les diméthicones copolyols comme décrit dans le document EP-A-970682.

Toutefois, si ces tensioactifs siliconés peuvent améliorer les propriétés cosmétiques des émulsions eau-dans-huile, ainsi que leur stabilité, le toucher des compositions les contenant reste caractéristique des silicones, c'est-à-dire glissant et peu émollient. Par ailleurs, dans une émulsion eau-dans-huile contenant un tensioactif siliconé, il est généralement nécessaire que les huiles de silicone constituent la majeure partie de la phase huileuse, ce qui limite le choix des constituants de la phase huileuse.

Par ailleurs, le document DE-A-19850758 décrit des émulsions inverses à forte teneur en eau à base d'un tensioactif polymérique tribloc dipolyhydroxyacyl/ polyalkylèneglycol de type Arlacel P135. Si ce type de tensioactif permet d'obtenir des émulsions fluides à forte teneur en eau, il ne garantit pas une stabilité suffisante, dans le temps et en température.

Il est également possible de réaliser des émulsions inverses à forte teneur en eau avec des phospholipides naturels tels que le décrivent les documents FR-A-2,777,180, FR-A-2,777,181, FR-A-2,777,194 et FR-A-2,777,195. Toutefois, la phase huileuse des compositions décrites dans ces documents est limitée à des mélanges restreints d'huiles. De plus, il est connu de l'homme de l'art que les phospholipides ont une stabilité limitée à l'oxydation et que celle-ci induit une déstabilisation du support les contenant.

Il subsiste donc le besoin d'obtenir une émulsion eau-dans-huile n'ayant pas les inconvénients de l'art antérieur, c'est-à-dire qui ait un toucher léger et frais, sans être siliconé, qui ait par ailleurs une teneur en phase aqueuse élevée et qui soit stable, même en l'absence d'agents structurants de la phase huileuse.

La demanderesse a maintenant trouvé de façon surprenante que les glycoglycérides permettaient de préparer des émulsions eau-dans-huile, riches en phase aqueuse et stables au stockage, présentant une consistance allant du lait fluide à la crème compacte.

Les glycoglycérides faisant l'objet de l'invention sont des esters comportant une ou deux chaînes alkyle et/ou alkényle et ils peuvent être obtenus par extraction ou par synthèse. Ceux obtenus par extraction sont extraits notamment de végétaux. Les produits de synthèse peuvent être obtenus en particulier par estérification d'un sucre comportant au moins une fonction acide carboxylique avec un monoglycéride ou un diglycéride. Les sucres concernés sont les oses (monosaccharides), oligoholosides (oligosaccharides) et les polyholosides (polysaccharides) fortement dépolymérisés dont la masse moléculaire est préférentiellement inférieure à 2000 g/mol. A titre d'exemples non limitatifs de ces sucres, on peut citer le glucose, le sucrose, le tréhalose, le sorbitol, le lactose, le fructose, le xylose, le maltose.

Les chaînes alkyle et/ou alkényle du glycoglycéride comportent de préférence de 10 à 22 atomes de carbone et sont choisies par exemple parmi les radicaux caprique, laurique, myristique, palmitique, stéarique, arachidique, béhénique, palmitoléique, oléique, linoléique, linolénique, gadoléique, arachidonique, érucique.

Selon un mode préféré de réalisation de l'invention, les glycoglycérides utilisés sont les galactolipides.

Certes, les glycoglycérides sont des composés connus. Ainsi, les documents EP-A-9842 et EP-A-249561 décrivent l'utilisation de galactolipides pour la préparation de liposomes, et le document WO-A-95/20945 décrit l'utilisation de digalactosyl diglycérides en association avec des lipides non polaires tels que le triacylglycérol, pour la préparation de supports lipophiles utilisables dans les domaines de la pharmacie, la cosmétique et l'agroalimentaire. Les supports décrits dans le document WO-A-95/20945 peuvent être des suspensions obtenues en ajoutant des quantités croissantes d'eau dans une phase huileuse contenant un digalactosyl-diglycéride. Toutefois, les émulsions eau-dans-huile décrites dans ce document contiennent de relativement faibles teneurs en phase aqueuse, bien inférieures à 80% du poids de la composition, avec des quantités importantes de galactolipides (20 %). Or, la demanderesse a trouvé de manière tout à fait inattendue qu'il était possible d'utiliser des galactolipides pour obtenir des émulsions eau-dans-huile riches en phase aqueuse et riches en eau, en vue d'obtenir des textures fraîches et légères, et ce avec des quantités en galactolipides bien inférieures à celles décrites dans le document WO-A-95/20945. Malgré le taux de phase aqueuse important (et la quantité importante d'eau) et la faible quantité de galactolipides, les émulsions E/H obtenues sont stables, ce qui est tout à fait surprenant. Rien ne laissait prévoir que l'on puisse obtenir de telles émulsions en utilisant des galactolipides.

L'invention a donc pour objet une composition comprenant, dans un milieu physiologiquement acceptable, une phase aqueuse dispersée dans une phase huileuse, caractérisée en ce qu'elle contient au moins un glycoglycéride et en ce que la phase aqueuse représente au moins 80 % en poids par rapport au poids total de la composition.

On entend par milieu physiologiquement acceptable dans la composition de l'invention, un milieu non toxique et susceptible d'être appliqué sur la peau (y compris l'intérieur des paupières), les cheveux, les ongles ou les lèvres d'êtres humains.

En dépit de la quantité importante de phase aqueuse, la composition de l'invention est stable dans le temps (bonne stabilité après 2 mois de 4°C à 45°C) ; elle ne déphase pas et ne relargue ni huile ni eau. En outre, elle apporte une très grande fraîcheur sur la peau.

La composition selon l'invention comporte au moins 80 % en poids de phase aqueuse par rapport au poids total de la composition, de préférence au moins 82 % et mieux 85 % du poids total de la composition. La phase aqueuse peut constituer jusqu'à 98 % du poids total de la composition. On entend ici par « phase aqueuse » tout ce qui constitue la phase dispersée, c'est-à-dire l'eau, les solvants et les additifs hydrosolubles ou hydrodispersibles. Comme solvants, on peut citer par exemple les mono-alcools linéaires ou ramifiés ayant de 1 à 8 atomes de carbone comme l'éthanol, le propanol, l'isopropanol, le butanol, l'isobutanol ; les polyéthylène glycols ayant de 4 à 80 oxydes d'éthylène ; les polyols comme la glycérine, le propylène glycol, l'isoprène glycol et le butylène glycol. Comme additifs hydrosolubles, on peut citer par exemple les sels inorganiques mono-, di- ou trivalents ; les filtres UV hydrosolubles tels que le Mexoryl SX, l'Eusolex 232 ; les agents de textures comme par exemple les gommes de xanthane, les dérivés cellulosiques, les guars, les carbomers, les dérivés acrylamides comme les polyacrylamides, les dérivés acryliques ; les sucres tels que le glucose, le sucrose, le tréhalose, le sorbitol, le lactose, le fructose, le xylose, le maltose Comme additifs hydrodispersibles, on peut citer par exemple les particules minérales ou organiques telles que les nanotitanes hydrodispersibles tels que le Mirasun TiW60 de la société Rhodia, le Tioveil AQ-N de la société Uniqema, les nano-oxydes de zinc, les argiles telles que la Laponite XLS de la société Laporte, les latex tels que l'Avalure AC-115 de la société Goodrich.

Selon un mode préféré de réalisation de l'invention, l'eau constitue au moins 70 %, de préférence au moins 75 % et mieux 80 % du poids total de la composition.

Comme glycoglycérides utilisables dans la composition de l'invention, on peut citer notamment les galactolipides et en particulier le digalactosyl diglycéride ou 1-O-[6-O-(α-D-galactopyranosyl)-β-D-galacto-pyranosyl]-2,3-di-O-acyl-D-glyceritol (DGDG) qui peut être sous forme hydrogénée ou non hydrogénée; le monogalactosyl diglycéride (MGDG) et leurs mélanges. Ces composés existent à l'état naturel dans les végétaux et plus particulièrement dans les membranes des végétaux. Ils peuvent être obtenus par extraction de ces végétaux, et par exemple à partir des céréales ou des épinards. Ils sont décrits par exemple dans les documents EP-A-9842 et EP-A-249561 incorporés ici pour référence. On peut utiliser notamment le DGDG et le MGDG fournis par les sociétés Serdary Research Laboratories ou Doosan. On peut aussi préparer le DGDG à partir de feuilles d'épinards fraîches, selon le procédé de l'exemple 1 du document EP-A-249561, ou bien de céréales selon le procédé de du document EP-A-9842.

Le DGDG a la structure (I) suivante : 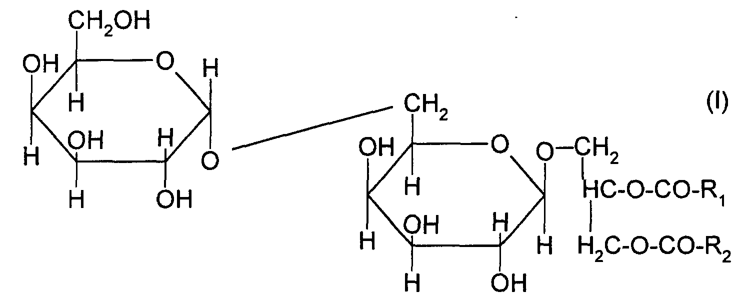 dans laquelle R₁ et R₂ peuvent être identiques ou différents et représentent un reste alkyle et/ou alkényl comportant de 10 à 22 atomes de carbone, tels que les radicaux caprique, laurique, myristique, palmitique, stéarique, arachidique, béhénique, palmitoléique, oléique, linoléique, linolénique, gadoléique, arachidonique, érucique.

On peut utiliser ces galactolipides purs ou dans des mélanges les contenant en des proportions plus ou moins importantes. On utilise de préférence des mélanges contenant au moins 10 % de DGDG par rapport aux lipides polaires du dit mélange.

La quantité de glycoglycéride(s) dans la composition de l'invention va de préférence de 0,1 à 10 % en poids, mieux de 0,1 à 5 % en poids et plus préférentiellement de 0,5 à 5 % en poids par rapport au poids total de la composition.

Les glycoglycérides sont de préférence introduits dans la phase huileuse de l'émulsion.

Les glycoglycérides peuvent être utilisés seuls ou en mélange avec des tensioactifs utilisés habituellement pour la préparation des émulsions eau-dans-huile. Comme tensioactifs utilisables, on peut citer par exemple les alkyl esters ou éthers de sorbitane, de glycérol ou de sucres, les alkyldiméthicone copolyols.

Lorsque la composition de l'invention contient des tensioactifs autres que les glycoglycérides, ces tensioactifs sont présents en une quantité allant de 0,1 à 50 % en poids par rapport au poids de glycoglycérides ou du mélange de lipides contenant les glycoglycérides.

La phase huileuse de la composition selon l'invention peut renfermer toute sorte d'huiles et de corps gras bien connus de l'homme du métier, comme exemple les huiles d'origine végétale (jojoba, avocat, sésame, tournesol, maïs, soja, carthame, pépins de raisin), les huiles minérales (vaseline, isoparaffines hydrogénées ou non hydrogénées, isohexadecane), les huiles de synthèse (parléam, myristate d'isopropyle, octanoate de cétéaryle, polyisobutylène, palmitate d'éthyl-hexyle, alkyl benzoates), les huiles de silicone volatiles ou non volatiles telles que les polydiméthylsiloxanes (PDMS) et les cyclodiméthylsiloxanes ou cyclométhicones, et les huiles fluorées ou fluorosiliconées, ainsi que les mélanges de ces huiles.

La phase huileuse peut contenir, en outre d'autres constituants gras tels que les alcools gras comme l'alcool stéarylique, l'alcool cétylique et l'alcool cétéarylique, les acides gras ; les cires ; les gommes de silicone ; les gélifiants huileux comme par exemple les alkylguars de la société Hercules ; les particules minérales ou organiques comme par exemple les oxydes de titane ou de nanotitane modifiés hydrophobes tels que le MT-100-Z de la société Tayca ; les pigments modifiés hydrophobes ; les particules de copolymères de méthacrylate de méthyle tels que le Polytrap 6035 de la société APS. La phase huileuse de la composition peut également contenir des filtres solaires organiques liposolubles tels que, par exemple, le Parsol 1789, le Parsol MCX, l'Uvinul N539, le Mexoryl XL.

Selon un mode particulier de réalisation de l'invention, la phase huileuse comprend au moins une huile choisie parmi les huiles minérales, les huiles de synthèse, les huiles de silicone volatiles et leurs mélanges, et notamment au moins une huile choisie parmi l'huile de parléam, l'isohexadécane, les cyclométhicones et leurs mélanges.

La quantité de phase huileuse dans la composition selon l'invention, en incluant la quantité de galactolipides, peut aller de 0,5 à 20 % en poids et de préférence de 2 à 18 % en poids par rapport au poids total de la composition.

Les compositions selon l'invention peuvent être plus ou moins fluides et elles peuvent donc se présenter sous forme de lait ou sous forme de crème plus ou moins épaisse. Ainsi, un autre avantage de la présente invention est de pouvoir obtenir des émulsions E/H de faible viscosité qui soient stables, émulsions qui sont particulièrement difficiles à obtenir. La viscosité des émulsions de l'invention peut donc varier dans une large mesure et aller notamment de 1 poise (0,1 Pa.s) à 150 poises (15 Pa.s), ces viscosités étant mesurées à environ 25°C à l'aide du viscosimètre "Rhéomat 180" qui est, de manière générale, équipé d'un mobile 2 pour les gammes de viscosités de 0,02 Pa.s à 0,7 Pa.s, d'un mobile 3 pour les gammes de viscosités de 0,2 Pa.s à 4 Pa.s, et d'un mobile 4 pour les gammes de viscosités de 2 Pa.s à 23 Pa.s.

Les compositions de l'invention sont préparées selon les méthodes usuelles de préparation d'une émulsion eau-dans-huile : après avoir préparé les phases aqueuse et huileuse, on incorpore progressivement et sous agitation, la phase aqueuse dans la phase huileuse comprenant les glycoglycérides. L'émulsion est généralement réalisée à une température allant d'environ 20 à 60°C.

La composition selon l'invention contient un milieu physiologiquement acceptable et trouve son application dans un grand nombre de traitements notamment cosmétiques de la peau, y compris du cuir chevelu, des cheveux, des ongles, et/ou des muqueuses. en particulier pour le soin, le nettoyage et/ou le maquillage et/ou la protection solaire de la peau, des cheveux et/ou des lèvres ou des muqueuses, ainsi que pour la préparation d'une crème destinée au traitement de la peau, plus particulièrement de la peau sèche du fait de la présence d'une phase huileuse externe.

Aussi, la présente invention a pour objet l'utilisation cosmétique de la composition telle que définie ci-dessus, pour le traitement, la protection, le soin, le démaquillage et/ou le nettoyage de la peau, des lèvres et/ou des cheveux, et/ou pour le maquillage de la peau et/ou des lèvres.

La présente invention a encore pour objet un procédé de traitement cosmétique de la peau, y compris du cuir chevelu, des cheveux, et/ou des lèvres, caractérisé par le fait que l'on applique sur la peau, les cheveux et/ou les lèvres, une composition telle que définie ci-dessus.

L'invention a aussi pour objet l'utilisation de la composition telle que définie ci-dessus pour la fabrication d'une crème destinée au traitement des peaux sèches.

De façon connue, la composition de l'invention peut contenir également des adjuvants habituels dans les domaines cosmétique et/ou dermatologique, tels que les électrolytes comme le sulfate de magnésium, les actifs, les conservateurs, les solvants, les parfums, les charges, les bactéricides, les absorbeurs d'odeur, les matières colorantes et encore les vésicules lipidiques. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse ou dans la phase aqueuse.

Comme actifs, on peut citer notamment, outre ceux indiqués plus haut, les hydratants et par exemple les hydrolysats de protéines et les polyols tels que la glycérine, les glycols comme les polyéthylène glycols, et les dérivés de sucre ; les extraits naturels ; les oligomères procyannidoliques ; les vitamines ; l'urée ; la caféine ; les dépigmentants tels que l'acide kojique et l'acide caféique ; les bêtahydroxyacides tels que l'acide salicylique et ses dérivés ; les alpha-hydroxyacides tels que l'acide lactique et l'acide glycolique ; les rétinoïdes tels que les caroténoïdes ; les filtres ; l'hydrocortisone ; la DHEA ; la mélatonine ; les extraits d'algues, de champignons, de végétaux, de levures, de bactéries ; les protéines hydrolysées, partiellement hydrolysées ou non hydrolysées, les enzymes et leurs mélanges.

Le ou les actifs peuvent être par exemple présents en une concentration allant de 0,01 à 20 %, de préférence de 0,1 à 5 % et mieux de 0,5 à 3 % du poids total de la composition.

Les exemples ci-après de compositions selon l'invention sont donnés à titre d'illustration et sans caractère limitatif. Les quantités y sont données en % en poids, sauf mention contraire.

### Exemple 1 : Lait hydratant

| *A*. *Phase huileuse* | |
|---|---|
| Galactolipides (DGDG fourni par la société Doosan) | 2,5 % |
| Isohexadécane | 7,7 % |
| Huile de parléam | 5,6 % |
| Cyclométhicone | 3,9 % |
| Conservateurs | 0,1 % |

| *B*. *Phase aqueuse* | |
|---|---|
| MgSO₄.7H₂O | 0,8 % |
| Eau | 78,6 % |
| Conservateurs | 0,6 % |

On obtient un lait blanc, ayant une viscosité équivalente de 14,9 Poises (1,49 Pa.s) (mobile 3, 200s⁻¹), stable au moins deux mois de 4 à 45°C.

### Exemple 2 : Crème peaux sèches

| *A. Phase huileuse* | |
|---|---|
| Galactolipides (DGDG fourni par la société Doosan) | 2,5 % |
| Isohexadécane | 2,9 % |
| Huile de parléam | 2,2 % |
| Cyclométhicone | 1,48 % |
| Conservateurs | 0,1 % |
| BHT (anti-oxydant) | 0,07 % |

| *B*. *Phase aqueuse* | |
|---|---|
| MgSO₄.7H₂O | 0,9 % |
| Eau | 88,5 % |
| Conservateurs | 0,6 % |

On obtient une crème souple, ayant une viscosité équivalente de 49,7 poises (4,97 Pa.s) (mobile 3, 200s⁻¹), stable au moins deux mois de 4 à 45°C.

## Revendications

1. Composition comprenant, dans un milieu physiologiquement acceptable, une phase aqueuse dispersée dans une phase huileuse, **caractérisée en ce qu'**elle contient au moins un glycoglycéride et **en ce que** la phase aqueuse représente au moins 80 % en poids par rapport au poids total de la composition.

2. Composition selon la revendication 1, **caractérisée en ce qu'**elle comporte au moins 70 % d'eau par rapport au poids total de la composition.

3. Composition selon la revendication 1 ou 2, **caractérisée en ce que** le glycoglycéride est obtenu par synthèse ou par extraction.

4. Composition selon l'une quelconque des revendications précédentes, **caractérisée en ce que** le glycoglycéride est extrait d'un végétal.

5. Composition selon l'une quelconque des revendications 1 à 3, **caractérisée en ce que** le glycoglycéride est obtenu par estérification d'un sucre comportant au moins une fonction acide carboxylique avec un monoglycéride ou un diglycéride.

6. Composition selon l'une quelconque des revendications précédentes, **caractérisée en ce que** le glycoglycéride est un galactolipide.

7. Composition selon la revendication précédente, **caractérisée en ce que** le galactolipide est choisi parmi le digalactosyl diglycéride hydrogéné ou non hydrogéné, le monogalactosyl diglycéride et leurs mélanges.

8. Composition selon l'une quelconque des revendications précédentes, **caractérisée en ce que** la quantité de glycoglycéride(s) va de 0,1 à 10 % en poids par rapport au poids total de la composition.

9. Composition selon l'une quelconque des revendications précédentes, **caractérisée en ce que** la phase aqueuse est présente en une quantité allant de 80 à 98 % en poids par rapport au poids total de la composition.

10. Composition selon l'une quelconque des revendications précédentes, **caractérisée en ce que** la phase huileuse est présente en une quantité allant de 0,5 à 20 % en poids par rapport au poids total de la composition.

11. Composition selon l'une quelconque des revendications précédentes, **caractérisée en ce que** la phase huileuse comprend au moins une huile choisie parmi les huiles minérales, les huiles de synthèse, les huiles de silicone volatiles et leurs mélanges.

12. Composition selon la revendication précédente, **caractérisée en ce que** la phase huileuse comprend au moins une huile choisie parmi l'huile de parléam, l'isohexadécane, les cyclométhicones et leurs mélanges.

13. Utilisation cosmétique de la composition selon l'une quelconque des revendications précédentes, pour le traitement, la protection, le soin, le démaquillage et/ou le nettoyage de la peau, des lèvres et/ou des cheveux, et/ou pour le maquillage de la peau et/ou des lèvres.

14. Procédé de traitement cosmétique de la peau, y compris du cuir chevelu, des cheveux, et/ou des lèvres, **caractérisé en ce que** l'on applique sur la peau, les cheveux et/ou les lèvres, une composition selon l'une quelconque des revendications 1 à 12.

15. Utilisation de la composition selon l'une quelconque des revendications 1 à 12 pour la fabrication d'une crème destinée au traitement des peaux sèches.
